# EUROPEAN PATENT APPLICATION

(11) **EP 2 206 481 A1**
(43) Date of publication of application: **14.07.2010**
(21) Application number: 10150184.9
(22) Date of filing: 06.01.2010
(51) Int. Cl.: A61F 2/00, A61F 5/41, A61H 19/00, A61N 1/05

(54) **Passive trainer for rehabilitating and treating incontinence**

(30) Priority: 07.01.2009 IL 19637409
(71) Applicant: Eliachar, Eliahu, 34371 Haifa (IL); Bressler, Eyal, 69018 Tel Aviv (IL); Sade Hochstadter, Dan, 10802 Bet Alfa (IL); Yossepowitch, Ofer, 49726 Petach Tikvah (IL); Baniel, Jack, 52246 Ramat-Gan (IL); Gabbay, Eli, 75507 Rishon Lezion (IL)
(72) Inventor: Eliachar, Eliahu, 34371, Haifa (IL); Sade Hochstadter, Dan, 10802, Bet Alfa (IL); Baniel, Jack, 52246, Ramat-Gan (IL); Gabbay, Eli, 75507, Rishon Lezion (IL)
(74) Representative: Lecomte, Didier

(57) **Abstract**

The present invention discloses a passive trainer for incontinence treatment and rehabilitation. The trainer comprises an elongated effecter (10) configured for being reversibly introduced entirely into the rectum via the anus; or into the vagina of a human patient; and, a stopper (20) coupled to said effecter, said stopper being adapted to remain external and adjacent to said anus, for preventing slippage of said effecter deeper into said rectum or said vagina; wherein the treatment and rehabilitation are obtained passively without any application of external forces.

## Description

### FIELD OF THE INVENTION

The present invention generally relates to a trainer for rehabilitating and improving continence and treating post-operative incontinence and methods thereof.

### BACKGROUND OF THE INVENTION

The present invention relates to a trainer for rehabilitating and improving continence and treating post-operative incontinence.

Urinary incontinence is a significant health concern worldwide. Urinary incontinence can be found in patients with neurological diseases such as Parkinson, CVA, Multiple Sclerosis. It is also found in elderly patients unable to control their sphincters due to weakness and lengthy treatment with steroids, in patients during and after chemotherapy treatments, in patients suffering from Irritable bowel syndrome, in patients with Constipation, in pregnant women, in patients with Osteoporosis, in athletes and after prostate operations.

In the urology field, needles, suture passers and ligature carriers are utilized in a variety of procedures, many of which are designed to treat incontinence. Moreover, various implantable devices, such as distensible medical devices, are known in which the distensible medical devices are implanted into the tissue of a human to treat urinary incontinence. These devices have typically relied upon restricting or constricting the urethra of the patient to maintain continence.

U.S. Patent No. 4,733,393 to Haber et al. is an attempt at such a proposed device. U.S. Patent No. 4,733,393 relates to a hypodermically implantable genitourinary prosthesis which provides an extensible, inflatable tissue expanding membrane to be located in proximal urethral tissue to add bulk to these tissues for overcoming urinary incontinence by localized increase in tissue volume.

U.S. Patent No. 4,802,479 to Haber et al. is an attempt at an instrument for dispensing and delivering material to an inflatable membrane of a genitourinary prosthesis within the tissues of a patient for overcoming urinary incontinence. U.S. Patent No. 4,832,680 to Haber et al. relates to an apparatus for hypodermically implanting a genitourinary prosthesis comprising an extensible containment membrane for retaining a fluid or particulate matter which is injected from an external source.

U.S. Patent No. 5,304,123 to Atala et al. relates to a detachable membrane catheter incorporated into an endoscopic instrument for implantation into the suburethral region of a patient. Also, U.S. Patent No. 5,411,475 to Atala et al. discusses a directly visualized method for deploying a detachable membrane at a target site in vivo.

Once inflated, these devices maintain pressure on the urethra of the patient in an attempt to assist with continence. However, these devices are prone to being under or over inflate at time of implant, leading to undesirable postoperative results. For example, if` the devices are overinflated it may cause the urethra to be restricted too tightly, and the patient is at risk for retention, a condition where the patient cannot pass urine. Such a condition could lead to kidney damage, necessitating major corrective surgery or at minimum use of self-catheterization to empty the bladder on a regular basis thus increasing the risk of urinary tract infection.

Furthermore, once these devices have been implanted within the patient, the only means of removing them in the event of a postoperative problem or device malfunction is through major surgery. Also, the devices are secured within the tissues of the patient, so there is the possibility of the devices migrating back along the pathway created in inserting them, a problem which has been noted with prior art devices.

Other patent literature relates to applying different stimulation to the anus's sphincter muscles and thus retraining the same. The most common stimulations used are electrical stimulation or mechanical vibration/oscillations.

As is commonly thought, brief doses of electrical stimulation can strengthen muscles in the lower pelvis in a way similar to exercising the muscles. Electrodes are temporarily placed in the vagina or rectum to stimulate nearby muscles. This can stabilize overactive muscles and stimulate contraction of urethral muscles.

An example of such teaching can be found in U.S. Patent No. 5,117,840 which provides an apparatus for restoring fecal continence to a patient. The apparatus comprises an electrical stimulation probe for insertion into the anus of a patient and associated electronics for producing a pulsatile waveform electrical output adapted for optimal physiological stimulation.

Such electrical stimulation usually causes a great deal of discomfort/pain to the patients and often does not achieve its goal. They do not actuate the muscles in a physiological manner, thus don't really "train" them. Additionally, the stimulus applied can injure the adjacent tissue - for example, electrical shocks and local heating or cooling can cause burns.

Since all such devices do not comply with a 'wireless' treatment, they usually restrict the patients both in time and location. The patients have to 'invest' the time needed for such treatment and even more importantly the patients are restricted to the clinic's location. In other words, non of the above mentioned devices is convenient to use in the comfort of home whilst the patients are fully mobilized.

Another major disadvantage common to all the above mentioned applications is the fact that all said devices are used whilst the patients are at least partially naked which results in some discomfort and distress to the patient. Therefore, it would be most advantageous to have a device which would enable its use while the patient is fully dressed.

Thus, there is a long felt need for an improved trainer for rehabilitating and treating urinary incontinence that would minimize the pain, injuries or any discomfort caused to the patients; would actuate the muscles in a physiological manner; and, would provide a wireless treatment such that the patients could remain fully dressed and mobilized.

### SUMMARY OF THE INVENTION

It is one object of the present invention to provide a passive trainer (100) for incontinence treatment and rehabilitation comprising:
a. an elongated effecter (10) configured far being reversibly introduced entirely into the rectum, via the anus or into the vagina of a human patient; and,
b. a stopper (20) coupled to said effecter (10), said stopper being adapted to remain external and adjacent to said anus, for preventing slippage of said effecter deeper into said rectum or said vagina;
wherein said treatment and rehabilitation are obtained passively without any application of external forces.

It is another object of the present invention to provide the passive trainer as defined above, wherein said trainer is adapted to provide said treatment and said rehabilitation by induction of continuous, constant and stable contraction of the pelvic levator muscles and/or anal sphincter and/or perivaginal smooth muscle of said patient for preventing outward slippage of said passive effecter.

It is another object of the present invention to provide the passive trainer as defined above, configured for being positioned in said rectum or said vagina for a period of at least about 2 hours.

It is another object of the present invention to provide the passive trainer as defined above, wherein said stopper is thin such that said stopper does not protrude past the outer surface of said anus or said vagina so as to provide said treatment and rehabilitation whilst said patient remains fully dressed.

It is another object of the present invention to provide the passive trainer as defined above, wherein said treatment and rehabilitation are obtained whilst said patient remains fully mobilized.

It is another object of the present invention to provide the passive trainer as defined above, wherein the shape of said effecter (10) is selected from a group consisting of curved, linear or any combination thereof.

It is another object of the present invention to provide the passive trainer as defined above, wherein said stopper comprising at least one handle (22) for facilitating extraction of said passive trainer.

It is another object of the present invention to provide the passive trainer as defined above, wherein said effecter dimensions (i.e., diameter and length) are varies from about 4 to 25 mm and from about 30 to 150 mm, respectively

It is another object of the present invention to provide the passive trainer as defied above, wherein said effecter comprising a penetration tip (**11**).

It is another object of the present invention to provide the passive trainer as defined above, wherein said effecter and/or said penetration tip is made of conventional elastic, resilient material, especially rubber, silicon rubber, natural latex or combination thereof.

It is another object of the present invention to provide the passive trainer as defined above, wherein said effecter is an elongated bladder characterized by an envelope and an inner portion comprising at least one compartment filled with flowing matter.

It is another object of the present invention to a kit comprising said trainer and disposable condom enveloping the same.

It is another object of the present invention to provide the passive trainer as defined above, wherein said flowing matter characterized by Shore A hardness of less than 5.

It is another object of the present invention to provide the passive trainer as defined above, wherein said flowing matter is selected from a group consisting of fluids, air, especially FDA approved gels, water, saline, glycerin, heat retaining materials, and flowing solids, especially fine particles, silica, silica gel, powders, aggregates or the like.

It is another object of the present invention to provide the passive trainer as defined above, wherein the wall thickness of said envelope is of at least 1 mm.

It is another object of the present invention to provide the passive trainer as defined above, wherein the effecter is of high tear propagation resistance having a Shore A hardness of about 30 to 70.

It is another object of the present invention to provide the passive trainer as defined above, wherein the stopper is constructed from a semi-rigid material, especially a polymer or high durometer silicon.

It is another object of the present invention to provide the passive trainer as defined above, wherein the penetration tip is constructed of a yieldable silicon material.

It is another object of the present invention to provide the passive trainer as defined above, wherein said handle is at least one hinged ring.

It is another object of the present invention to provide the passive trainer as defined above, wherein said handle is at least one inset beveled slot.

It is another object of the present invention to provide the passive trainer as defined above, wherein said effecter comprising means adapted to facilitate the passage of fluids, medicaments, accessories, especially optic fibers, and surgical tools.

It is another object of the present invention to provide the passive trainer as defined above, wherein said effecter comprising means adapted to facilitate the passage of diagnostic sensors into the patient body cavity, said sensors interconnected by wire or wirelessly with diagnostic apparatus.

It is another object of the present invention to provide the passive trainer as defined above, wherein said bladder is perforated to facilitate controlled release of substances towards the rectal wall.

It is another object of the present invention to provide the passive trainer as defined above, wherein said substances are selected from a group consisting of fluids, medicaments, pastes, gels, lotions, creams, salves or combination thereof.

It is another object of the present invention to provide the passive trainer as defined above, useful for removal of fecal samples especially for use in occult fecal blood examinations comprising of a plurality of protuberances located at the outer portion of the shaft and/or penetration tip[D S H1].

According to another embodiment of the present invention, the passive trainer as defined above, is hollow and contains an opening along both edges of said trainer, such that an absorbing member such as a tampon can be inserted along at least part of its length.

It is another object of the present invention to provide the passive trainer as defined above, wherein at least one of said protuberances additionally comprising heating/cooling means.

It is another object of the present invention to provide the passive trainer as defined above, wherein said heating/cooling can be provided by means selected from a group consisting of Pelletier device, electrical condenser, heated electrical element, pre-cooled or pre-heated materials or a combination thereof.

It is another object of the present invention to provide the passive trainer as defined above, wherein said passive trainer is hollow and contains an opening along both edges of said trainer, such that an absorbing member such as a tampon can be inserted along at least part of its length.

It is another object of the present invention to provide the passive trainer as defined above, wherein the shape of said effecter is curvature especially adapted to treat a male patient.

It is another object of the present invention to provide the passive trainer as defined above, wherein the shape of said effecter is curvature especially adapted to treat a female patient.

It is another object of the present invention to provide the passive trainer as defined above, wherein said effecter comprises heating/cooling means.

It is another object of the present invention to provide a heating/cooling trainer, wherein said heat/cool is provided by means selected from a group consisting of Pelletier device, electrical condenser, heated electrical clement, pre-cooled or pre-heated materials or a combination thereof.

It is another object of the present invention to provide the passive trainer as defined above, wherein said penetration tip additionally comprising heating/cooling means.

It is another object of the present invention to provide the passive trainer as defined above, wherein said heating/cooling can be provided by means selected from a group consisting of Pelletier device, electrical condenser, heated electrical element, pre-cooled or pre-heated materials or a combination thereof.

It is another object of the present invention to provide the passive trainer as defined above, additionally comprising a stimulation mechanism adapted to actively induce outward slippage of said passive trainer, such that a more effective contraction of the pelvic levator muscles and/or anal sphincter and/or perivaginal smooth muscle of said patient is obtained.

It is another object of the present invention to provide the passive trainer as defined above, wherein said stimulation mechanism is be selected from a group consisting of any external forces such as electrical, thermal, mechanical (e.g., vibrations, oscillation or any other massaging effect), magnetic, introduction of any kind of fluids or any combination thereof.

It is another object of the present invention to provide the passive trainer as defined above, wherein said stimulation mechanism is randomly operated.

It is another object of the present invention to provide the passive trainer as defined above, wherein said stimulation mechanism is actively operated.

It is another object of the present invention to provide the passive trainer as defined above, wherein said stimulation mechanism is pre- programmed to operate according to a predetermined protocol

It is another object of the present invention to provide a method of rehabilitating and treating incontinence. The method comprises steps selected inter alia from:
a. obtaining a passive trainer **(100)** for incontinence treatment and rehabilitation comprising;
   i. an elongated effecter (**10**); and,
   ii. a stopper (**20**) coupled to said effecter (**10**);
b. reversible introducing said effector into the rectum, via the anus or into the vagina of a human patient whilst leaving said stopper external and adjacent to said anus, thereby preventing slippage of said effector deeper into said rectum or said vagina;
c. preventing outward slippage of said passive trainer by constantly, stably and continuously contracting the rectal muscles of said patient, thereby rehabilitating and treating incontinence;
wherein said step of constantly contracting the pelvic levator muscles and anus's muscles is performed without any application of external force by said passive trainer.

It is another object of the present invention to provide the method as defined above, wherein said step of reversibly introducing said effecter into the rectum or into said vagina is performed for a period of at least about 2 hours.

It is another object of the present invention to provide the method as defined above, wherein said step of rehabilitating and treating incontinence is performed whilst said patient remains fully dressed.

It is another object of the present invention to provide the method as defined above, wherein said step of rehabilitating and treating incontinence is performed whilst said patient remains fully mobilized.

It is another object of the present invention to provide the method as defined above, additionally comprising step of selecting the shape of said effecter (10) from a group consisting of curved, linear or any combination thereof.

It is another object of the present invention to provide the method as defined above, additionally comprising step of heating and/or cooling the effecter to a predetermined temperature, ranging from 4 to 42 degrees Celsius.

It is another object of the present invention to provide the method as defined above, additionally comprising step of administrating fluids, medicament, accessories, especially optic fibers and surgical tools while said effected is deployed within the patient's body cavity.

### BRIEF DESCRIPTION OF THE DRAWING

The objects and advantages of various embodiments of the invention will become apparent from the following description when read in conjunction with the accompanying drawings w-herein:
FIG. 1 schematically illustrating a trainer according to one embodiment of the invention.
FIGS. 2 and 3 illustrates a top view and side view of trainer 100.
FIGS. 4 and 5 front view of two embodiments of the invention, i.e., two handles (FIG. 4) and one handle (FIG. 5) versions.

### DETAILED DESCRIPTION OF THE INVENTION

The following description is provided, alongside all chapters of the present invention, so as to enable any person skilled in the art to make use of said invention and sets forth the best modes contemplated by the inventor of carrying out this invention. Various modifications, however, will remain apparent to those skilled in the art, since the generic principles of the present invention have been defined specifically to provide a device and method for treating incontinence treatment and rehabilitation.

The present invention provides a passive trainer **(100)** for incontinence treatment and rehabilitation comprising (a) an elongated effecter (**10**) being reversibly introduced entirely into the rectum, via the anus of a human patient; and, (b) a stopper **(20)** coupled to said effecter (10), said stopper is adapted to remain external and adjacent to said anus, for preventing slippage of said effected deeper into said rectum. The treatment and rehabilitation are obtained passively without any application of external forces.

Alternatively the passive trainer (**100**) can be inserted into the vagina.

The term **"external forces"** refers hereinafter to any kind of stimulation or irrigation that can be applied to the anus's muscles such as electrical, thermal, mechanical (cm;., vibrations), magnetic, introduction of any kind of fluids or any combination thereof.

The term **"treatment time"** refers hereinafter to the amount of time each single treatment requires.

The term **"about"** refers hereinafter to a range of 25% below or above the referred value.

The term **"stable"** refers hereinafter to a constant and continues pressure applied on the muscles by the passive trainer. This is in sharp contrast to other active trainer which, flour example, applies vibration on the muscles. During such stimulation, a time-varied forces is applied on the muscles. One object of the present invention is to apply a constant (i.e., stable) force on the muscle.

It should be emphasized that the main advantages of the passive trainer are the ability to remain within the patient's body for long periods of time (i.e., several hours) without causing major discomfort or pain.

According to some embodiments, the treatment is performed on a daily basis, in which the patient can continue its daily routine while the passive trainer is in use.

Furthermore, the incontinence treatment and rehabilitation can be obtained while the patient is fully dressed such that any additional embarrassment that usually accompanies this kind of treatment is avoided.

Yet more, the passive trainer provided by the present invention enables rehabilitation and treatment whilst the patients are fully mobilized and continue their daily routine without any interruptions.

According to other embodiments, different rehabilitation programs can be adjusted. Said programs can be selected from muscles endurance.

The passive trainer (**100**) of the present invention comprises (a) an elongated effecter (**10**) being reversibly introduced entirely into the rectum, via the anus of a human patient; and, (b) a stopper (**20**) coupled to said effecter (**10**), said stopper is adapted to remain external and adjacent to said anus, for preventing slippage of the effecter deeper into said rectum. The treatment and rehabilitation are obtained passively without any application of external forces.

According to a preferred embodiment of the present invention, the incontinence treatment and rehabilitation is obtained by introducing the effecter (**10**) into the rectum, via the anus of the patient; and, leaving the stopper externally and adjacent to the anus, for preventing slippage of the effecter deeper into the rectum.

In order to prevent slippage of the effector from outside the body, the patient constantly contracts the anus's sphincter muscles and by doing so, the muscles are rehabilitated. Since the perineal muscles are intertwined with the rental sphincter muscles, the trainer strengthens simultaneously also other muscles, such as the external urethral sphincter, thus treating urinary incontinence in addition to fecal incontinence.

It should be emphasized that the treatment and rehabilitation are obtained passively without any application of external forces.

It should be noted that the passive trainer (**100**) can be alternatively inserted into the vagina.

It is in the scope of the invention wherein a trainer (**100**) for rehabilitating and improving continence and treating post-operative incontinence is disclosed. The trainer comprises of (i) an elongated effecter (**10**) suitable for being reversibly introduced into the rectum, via the anus of a human patient; said effecter optionally comprising a penetration tip (**11**); and, (ii) handle (**20**) comprising a stopper (**21**) preventing slippage of said effecter into said body cavity; and optionally a at least one handle (**22**).

It is further in the scope of the invention wherein the aforesaid effecter is of dimensions (i.e., diameter and length) that vary from about 4 to 25 mm and from about 30 to 150 mm, respectively.

Alternatively, it should be noted that the passive trainer (**100**) can be inserted into the vagina.

According to another embodiment of the present invention, the effecter's diameter at the area encompassed by the rectal sphincter can be varied and optimized during the course of the rehabilitation process. During the course of the training, the perineal muscles strengthen and contract, thus, a smaller effecter's diameter can be used.

According to another embodiment of the present invention, other features of the effecter can also be adjusted to the stage of rehabilitation and muscular strength achieved. The features that can be varied can be the effecter's toughness, composition, roughness of surface and shape.

The toughness or roughness degree can be noticed and distinguished by means such as coloring. For example, each toughness or roughness degree can be in a different color.

It is further in the scope of the invention wherein the said effector and/or said penetration tip is made of conventional elastic or semi elastic, resilient material, especially rubber, silicon rubber, natural latex or alike biocompatible materials or combinations thereof.

It is further in the scope of the invention wherein the said effecter is an elongated bladder characterized by an envelope and an inner portion comprising at least one compartment filled with flowing matter.

It is further in the scope of the invention wherein the said effecter is included in a kit comprising of said trainer and disposable condom enveloping the same.

It is further in the scope of the invention wherein said flowing matter characterized by Shore A hardness of less than 5.

It is further in the scope of the invention wherein said flowing matter is selected from a group consisting of fluids, air, especially FDA approved gels, water, saline, glycerin, heat retaining materials, and flowing solids, especially fine particles, silica, silica gel, powders, aggregates or the like.

It is further in the scope of the invention wherein the wall thickness of said envelope is of at least 1 mm.

It is further in the scope of the invention wherein the effecter is of high tear propagation resistance having a Shore A hardness of about 30 to 70.

It is further in the scope of the invention wherein the stopper is constructed from a semi-rigid material, especially a polymer or high durometer silicon or alike.

It is further in the scope of the invention wherein the penetration tip is constructed of a yieldable silicon material.

It is further in the scope of the invention wherein said handle is at least one hinged ring.

It is further in the scope of the invention wherein said handle is at least one inset beveled slot.

It is further in the scope of the invention wherein said effecter comprising means to facilitate the passage of fluids, medicaments, accessories, especially optic fibers and surgical tools.

It is further in the scope of the invention wherein said effecter comprising means to facilitate the passage of diagnostic sensors into the patient body cavity, said sensors interconnected by wire or tirelessly with diagnostic apparatus.

It is further within the scope of the invention wherein an applicator is provided, including a bladder, perforated to facilitate controlled release of substances towards the rectal wall.

It is further within the scope of the invention wherein said applied substances are selected from a group consisting of fluids, medicaments, pastes, gels, lotions, creams, salves or combination thereof.

It is further within the scope of the invention wherein an extractor is provided, useful for removal of` fecal samples especially for use in occult fecal blood examinations comprising of a plurality of protuberances located at the outer portion of the shaft and/or penetration tip.

According to some embodiments at least one of said protuberances comprises heating/cooling means. The heat/cool can be provided by means selected from Pelletier device, electrical condenser, heated electrical element, pre-cooled or pre-heated materials or a combination thereof.

According to some embodiments only the penetration tip (11) of the effecter comprises heating/cooling means. The heat/cool can be provided by means selected from Pelletier device, electrical condenser, heated electrical element, pre-cooled or pre-heated materials or a combination thereof.

It is further in the scope of the invention wherein said effecter comprising means to facilitate the passage of fluids, medicaments, accessories, especially optic fibers and surgical tools.

It is further in the scope of the invention wherein said trainer is appropriately curved and adapted to treat a male patient. Alternatively, it is in the scope of the invention wherein said trainer is appropriately curved and adapted to treat a female patient.

It is further in the scope of the invention to disclose a method of rehabilitating and treating post-operative incontinence. The method comprises of (i) obtaining a trainer as defined in any of the above, and (ii) reversibly introducing said effecter into the rectum, via the anus of a human patient.

Alternatively, it should be noted that the passive trainer (**100**) can be inserted into the vagina.

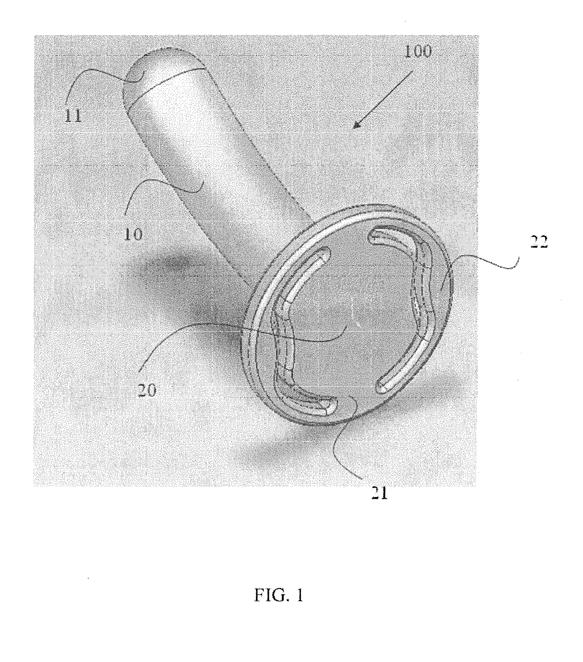

Reference is now made to FIG. 1, schematically illustrating the passive trainer according to one embodiment of the invention. The trainer comprises of an elongated and curved shaft (effecter 10) with a penetration tip (11). The shaft is at least temperately attached to stopper 20 having a defined rim 21. The stopper comprises of two handles 22.

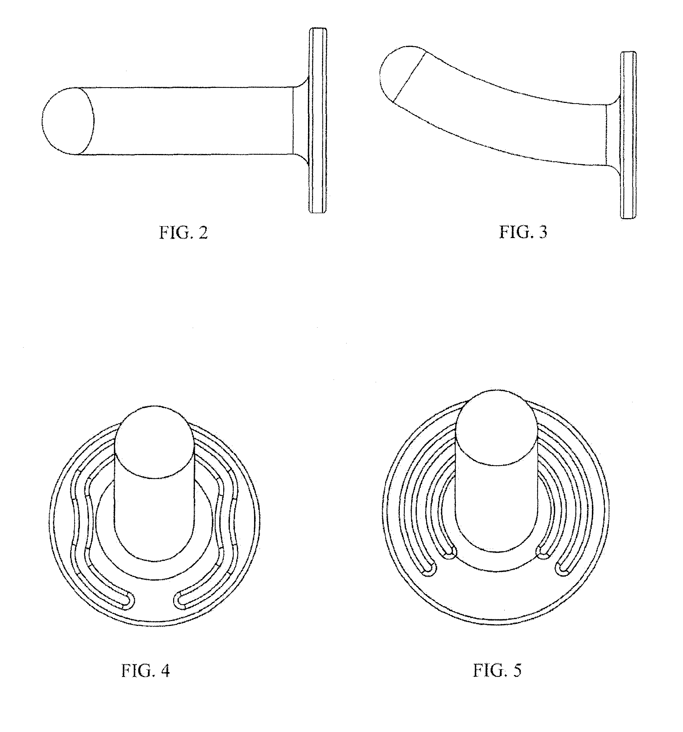

Reference is now made to FIGS. 2 and 3, illustrating a top view and side view of trainer 100. FIGS. 4 and 5 front view of two embodiments of eth invention, i.e., two handles (FIG. 4) and one handle (FIG. 5) versions.

According to another embodiment of the present invention the passive trainer as defined above is adapted to provide said treatment and said rehabilitation by induction of continuous contraction of the rectal muscles of said patient for preventing outward slippage of said passive effecter.

According to another embodiment of the present invention the passive trainer as defined above is configured for being positioned in said rectum/vagine for a period of at least about 2 hours. However, it should be emphasized that the passive trainer may be retained with the rectum/vagine at a period of a couple of says, or even weeks.

According to some embodiments, the treatment is performed on a daily basis, in which the patient can continue its daily routine while the passive trainer is in use.

According to other embodiments, different rehabilitation programs can be adjusted. Said programs can be selected from muscles endurance.

According to another embodiment of the present invention the stopper is thin such that said stopper does not protrude past the outer surface of said anus or said vagina so as to provide said treatment and rehabilitation whilst said patient remains fully dressed.

According to another embodiment of the present invention the treatment and rehabilitation are obtained whilst said patient remains fully mobilized.

According to another embodiment of the present invention the shape of the effecter (10) is selected from a group consisting of curved, linear or any combination thereof.

According to another embodiment of the present invention the stopper comprising at least one handle (**22**) for facilitating extraction of said passive trainer.

According to another embodiment of the present invention the effecter's dimensions (i.e., diameter and length) are varies from about 4 to 25 mm and from about 30 to 150 mm, respectively

According to another embodiment of the present invention the effecter and/or the penetration tip is made of conventional elastic, resilient material, especially rubber, silicon rubber, natural latex or combination thereof.

According to another embodiment of the present invention the effecter is an elongated bladder characterized by an envelope and an inner portion comprising at least one compartment filled with flowing matter.

According to another embodiment of the present invention a kit comprising of said trainer and disposable condom enveloping the same is provided.

According to another embodiment of the present invention the flowing matter characterized by Shore A hardness of less than 5.

According to another embodiment of the present invention the flowing matter is selected from a group consisting of fluids, air, especially FDA approved gels, water, saline, glycerin, heat retaining materials, and flowing solids, especially fine particles, silica, silica gel, powders, aggregates or the like.

According to another embodiment of the present invention the wall thicknesses of said envelope is of at least 1, mm.

According to another embodiment of the present invention the effecter is of high tear propagation resistance having a Shore A hardness of about 30 to 70.

According to another embodiment of the present invention the stopper is constructed from a semi-rigid material, especially a polymer or high durometer silicon.

According to another embodiment of the present invention the penetration tip is constructed of a yieldable silicon material.

According to another embodiment of the present invention the handle is at least one hinged ring.

According to another embodiment of the present invention the handle is at least one inset beveled slot.

According to another embodiment of the present invention the effecter comprising means adapted to facilitate the passage of fluids, medicaments, accessories, especially optic fibers, and surgical tools.

According to another embodiment of the present invention the comprising means adapted to facilitate the passage of diagnostic sensors into the patient body cavity, said sensors interconnected by wire or wirelessly with diagnostic apparatus.

According to another embodiment of the present invention the bladder is perforated to facilitate controlled release of substances towards the rectal wall.

According to another embodiment of the present invention the substances are selected from a group consisting of fluids, medicaments, pastes, gels, lotions, creams, salves or combination thereof.

According to another embodiment of the present invention the passive trainer is useful for removal of fecal samples especially for use in occult fecal blood examinations comprising of a plurality of protuberances located at the outer portion of the shaft and/or penetration tip.

According to another embodiment of the present invention the shape of said effecter is curvature especially adapted to treat a male patient.

According to another embodiment of the present invention the shape of said effecter is curvature especially adapted to treat a female patient.

According to another embodiment of the present invention the effecter comprises of heating/cooling means.

According to another embodiment of the present invention a heating/cooling trainer is provided. The heat/cool is provided by means selected from Pelletier device, electrical condenser, heated electrical element, pre-cooled or pre-heated materials or a combination thereof.

According to another embodiment of the present invention a method of rehabilitating and treating incontinence is provided. The method comprising steps selected inter alia from:
a. obtaining a passive trainer (100) for incontinence treatment and rehabilitation comprising;
   i. an elongated effecter (**10**); and,
   ii. a stopper (**20**) coupled to said effecter (**10**);
b. reversibly introducing said effecter into the rectum, via the anus of a human patient whilst leaving said stopper external and adjacent to said anus, thereby preventing slippage of said effecter deeper into said rectum;
c. preventing outward slippage of said passive trainer by constantly, stably and continuously contracting the rectal muscles of said patient, thereby rehabilitating and treating incontinence;
wherein said step of constantly contracting the anus's muscles is performed without any application of external force by said passive trainer.

Alternatively, it should be noted that the passive trainer (**100**) can be inserted into the vagina.

According to another embodiment of the present invention the method as described above, wherein said step of reversibly introducing said effecter into the rectum/vagina is performed for a period of at least about 2 hours.

According to another embodiment of the present invention the method as described above, wherein said step of rehabilitating and treating incontinence is performed whilst said patient remains fully dressed.

According to another embodiment of the present invention the method as described above, wherein said step of rehabilitating and treating incontinence is performed whilst said patient remains fully mobilized.

According to another embodiment of the present invention the method as described above, additionally comprising step of selecting the shape of said effecter (10) from a group consisting of curved, linear or any combination thereof.

According to another embodiment of the present invention the method as described above, additionally comprising step of heating and/or cooling the effector to a predetermined temperature, ranging from 4 to 42 degrees Celsius.

According to another embodiment of the present invention the method as described above, additionally comprising step of administrating fluids, medicaments, accessories, especially optic fibers and surgical tools while said effecter is deployed within the patient's body cavity.

According to another embodiment of the present invention, the passive trainer as defined above, additionally comprising stimulation mechanism adapted to actively induce outward slippage of said passive trainer, such that a more effective contraction of the pelvic levator muscles and/or anal sphincter and/or perivaginal smooth muscle of said patient is obtained. According to another embodiment of the present invention, the stimulation mechanism can be selected from a group consisting of any external forces such as electrical, thermal, mechanical (e.g., vibrations, oscillation or any other massaging eftect), magnetic, introduction of any kind of fluids or any combination thereof

According to another embodiment of the present invention, the stimulation mechanism can be actively or randomly operated. Alternatively it can be pre-programmed to operate according to a predetermined protocol.

## Claims

1. A passive trainer (**100**) for incontinence treatment and rehabilitation comprising:
a. an elongated effecter (**10**) configured for being reversibly introduced entirely into the rectum via the anus; or into the vagina of a human patient; and,
b. a stopper (**20**) coupled to said effecter (**10**), said stopper being adapted to remain external and adjacent to said anus, for preventing slippage of said effecter deeper into said rectum or said vagina;
wherein said treatment and rehabilitation are obtained passively without any application of external forces.

2. The passive trainer according to claim 1, wherein said trainer is adapted to provide said treatment and said rehabilitation by induction of continuous and stable contraction of the pelvic levator muscles and/or anal sphincter and/or perivaginal smooth muscle of said patient for preventing outward slippage of said passive effecter.

3. The passive trainer according to claim 1, additionally comprising a stimulation mechanism adapted to actively induce outward slippage of said passive trainer, such that a more effective contraction of the pelvic levator muscles and/or anal sphincter and/or perivaginal smooth muscle of said patient is obtained; said stimulation mechanism is selected from a group consisting of any external forces such as electrical, thermal, mechanical (e.g., vibrations, oscillation or any other massaging effect), magnetic, introduction of any kind of fluids or any combination thereof.

4. The passive trainer according to claim 3, wherein said stimulation mechanism is either randomly or actively operated.

5. The passive trainer according to claim 1, wherein the shape of said effecter (10) is selected from a group consisting of curved, linear or any combination thereof.

6. The passive trainer according to claim 1, wherein said stopper comprising at least one handle (**22**) for facilitating extraction of said passive trainer.

7. The passive trainer according to claim 1, wherein said effecter comprising a penetration tip (**11**).

8. The passive trainer according to claim 1, wherein said effector is an elongated bladder **characterized by** an envelope and an inner portion comprising at least one compartment filled with flowing matter.

9. The passive trainer according to claim 1, wherein said effecter is of high tear propagation resistance having a Shore A hardness of about 30 to 70.

10. The passive trainer according to claim 1, wherein said effecter comprising means adapted to facilitate the passage of diagnostic sensors into the patient body cavity, said sensors interconnected by wire or wirelessly with diagnostic apparatus.

11. The passive trainer according to claim 1, useful for removal of fecal samples especially for use in occult fecal blood examinations comprising of a plurality of protuberances located at the outer portion of the shaft and/or penetration tip.

12. The passive trainer according to claim 1, wherein said effecter comprises heating/cooling means.

13. A kit comprising the passive trainer as defined in claim 1 and disposable condom enveloping the same; said flowing matter **characterized by** Shore A hardness of less than 5.
